Europäisches Patentamt

(19) European Patent Office

Office européen des brevets

(11) Publication number: **0 103 031**
**B1**

(12) **EUROPEAN PATENT SPECIFICATION**

(45) Date of publication of patent specification: **07.05.86**

(21) Application number: **82108135.3**

(22) Date of filing: **03.09.82**

(51) Int. Cl.⁴: **G 01 N 27/30, G 01 N 27/40**

(54) **Electro-chemical cell for determining a particular property or component of a fluid.**

(43) Date of publication of application:
**21.03.84 Bulletin 84/12**

(45) Publication of the grant of the patent:
**07.05.86 Bulletin 86/19**

(84) Designated Contracting States:
**CH DE FR GB IT LI NL SE**

(56) References cited:
**US-A-2 913 386**
**US-A-3 856 649**
**US-A-4 053 381**
**US-A-4 214 968**

(73) Proprietor: **HONEYWELL MEDICAL
ELECTRONICS B.V.
I.B.C.-Weg 1
NL-5683 PK Best (NL)**

(72) Inventor: **Kuypers, Martinus Henricus
Schoolstraat 53
NL-5561 AH Riethoven (NL)**

(74) Representative: **Rentzsch, Heinz et al
Honeywell Europe S.A. Holding KG Patent- und
Lizenzabteilung Kaiserleistrasse 55
D-6050 Offenbach am Main (DE)**

## Description

The invention relates to an electro-chemical cell in accordance with the preamble of claim 1. A cell of this type is shown in US—A—40 53 381 and is used for determining the ion activity in drops of biological fluids. A drop of a reference solution and a drop of a test solution are applied to two locations spaced along a porous bridge layer consisting of polyamide or polycarbonate. The two solutions migrate along the bridge layer and the electrical potential between electrodes provided in electrical contact with said locations is measured. An ion-selective membrane covers an internal reference element consisting of a metal layer, a metal salt layer and an electrolyte solution layer, which preferably is a dried hydrophilic layer comprising a hydrophilic binder and a suitable ionic salt.

US—A—38 56 649 describes a solid state electrode for use in determining ion concentration in an aqueous solution. The electrode comprises an electrically conductive inner element with a salt disposed on a surface portion thereof having as a cation a cation form of the element and also having an anion, a hydrophilic layer in intimate contact with the salt and including a water soluble salt of said anion and a hydrophobic layer in intimate contact with the hydrophilic layer whereby the hydrophilic layer is shielded from contact with the ion-containing aqueous solution. This electrode may function as a reference electrode as well as an electrode for identifying specific ions.

A further ion-selective electrode is shown in US—A—42 14 968 and comprises a dried, internal reference electrode, and in contact with the reference electrode, a hydrophobic ion-selective membrane. The reference electrode may comprise either a metal/-metal salt reference half-cell or multiple-layer redox couple reference electrode. The hydrophobic membrane includes a binder having dispersed therein a solution of an ion carrier dissolved in a suitable carrier solvent.

Another type of electro-chemical cells is the so-called Clark Cell used for measurements in biological media. For this purpose reduction of the dimensions of such cells is desirable, in particular for measuring inside blood vessels. Since the metal of the anode as well as the electrolyte are consumed by the electro-chemical process during oxygen detection, a reasonable amount of both materials must be available in the cell. This is one of the limits for reducing the size of a Clark Cell. The required amount of electrolyte depends on the desired sensitivity of the sensor. This in turn is a function of the dimensions of the cathode and the thickness and permeability of the membrane. In most cases a small diameter of the cathode is favorable for obtaining a high-quality measuring signal in order to keep this signal uneffected by the flow conditions of the biological medium. If the resulting electrical signal, however, is too small, interference and noise signals will disturb the reliability of the measuring signal.

The use of small cathodes formed as a circle, spiral or line results in a high electrical current. A high current consumes a relatively large amount of electrolyte which has to be stored in the cell. The combination of an optimum shape and extension of the cathode and the required miniaturization of the sensor, therefore, require a compromise.

The electrolyte which is necessary for the electrochemical process in a Clark Cell is in a sufficient amount available in the biological medium. In the conventional Clark Cell one of the functions of the membrane is to define the oxygen flux to the cathode. The other function of the membrane is to separate the electrolyte in the Clark Cell from the electrolyte in the biological medium in order to avoid mixing of the two electrolytes. A direct contact between the biological medium and the cathode of the Clark Cell further would reduce the functionality of the cell because of adhesion of proteins to the surface of the cathode which would reduce the effective surface area of the cathode.

A second type of the Clark Cell is known using a porous membrane made of methylcellulose, polystyrene or a hydrophylic polymer such as HEMA (hydroxyethylmetacrylate), which is simultaneously used for defining the oxygen flux on the one side and to allow the transport of the electrolyte from the biological medium into the Clark Cell. One advantage of this type of cell is the possibility to store the dry sensor over a long period. The electrolyte enters the cell as soon as it is poured into contact with the biological medium. A disadvantage of this cell, however, is that the membrane may be deformed or swell in an undefined manner which alters the oxygen flux and therewith the sensitivity of the cell. This type of a Clark Cell, therefore, has to be recalibrated relatively often.

In a third type of Clark Cell the necessary amount of electrolyte is stored as dry salt under the membrane so that this cell can be stored in dry form over a long period of time. Activation of the cell is accomplished as soon as water vapor migrates through the membrane and dissolves the salt, therewith forming the liquid electrolyte. However, for this type of Clark Cell a long activation period of about four hours is required before the cell becomes fully operable.

It is an object of the invention to provide a new electrochemical cell of the type as defined above in which the electrolyte is supplied by the fluid under measurement without the danger that deformations of the membrane change the sensitivity of the cell in a non-controlled manner. The new cell should be capable of being miniaturized and should be suitable for medical and biological application, i.e. sterilization should be no problem. These objects and further improvements are accomplished by the invention as outlined in claim 1. The provision of a known porous hydrophobic membrane having holes at defined locations allows passage of the electrolyte into and through the hydrophylic polymer only at those locations from where the electrolyte moves into the space between the electrodes. The hydrophylic polymer is sufficiently permeable for the electrolyte in the biological medium, but its pores are small enough not to allow biological molecules such as proteins to pass. The hydrophylic polymer, therefore, is almost impermeable for proteins. There is no danger of poisoning the cathode. On the other side it is important that the oxygen diffusion through the holes in the membrane and via the capillaries in the hydrophylic polymer to the cathode is essentially smaller than the oxygen diffusion through the membrane. This is accomplished by locating the holes far enough from the cathode, with other words the distance between the holes and the cathode is relatively large as compared to the width of the cathode. The cathode can be made circular, spiral or line-shaped and could have a width of 5 μ and a length of 1000 μ. This cell can be stored sterile and dry over a long period of time.

The hydration and polarization of the cell is accomplished rather quickly. Within a few minutes after inserting the sensor into a calibration liquid or into the bloodstream the sensor is operable and gives a stable signal. The lifetime of the sensor is determined by the amount of anode material, for instance silver. For measuring the arterial oxygen content it may be supplied with a sufficient amount for reaching a life-time of several days. The silver anode may be covered by the hydrophylic polymer layer or it can be located externally on the system, consisting of the cathode, the hydrophylic polymer layer and the membrane.

A further advantage of the hydrophylic polymer layer is that it can be used for immobilizing biochemically active elements. In known Clark Cells for sensing glucose the glucoseoxidase enzyme is used. This enzyme can only be reached by molecules which penetrate the hydrophylic polymer so that proteins do not reach the enzyme if it is situated close to the electrodes.

The new electro-chemical cell can also be used as an ion-selective sensor for measuring the pH value of a medium. In this case an ion-selective membrane is covered by the hydrophylic polymer and a hydrophobic membrane provided with holes so that the ion-selective membrane is sufficiently protected against the blood and in fact, is only in contact with the water-phase of the blood and smaller biochemical molecules.

Further preferred modifications and improvements of the invention are described in the subclaims. The invention will be described with respect to two embodiments schematically shown in the drawings, whereat

Figure 1 shown an oxygen sensor of the Clark type and

Figure 2 shows a modification where the sensor is formed as a monolithic sensor manufactured in thin or thick film technology on an insulating substrate.

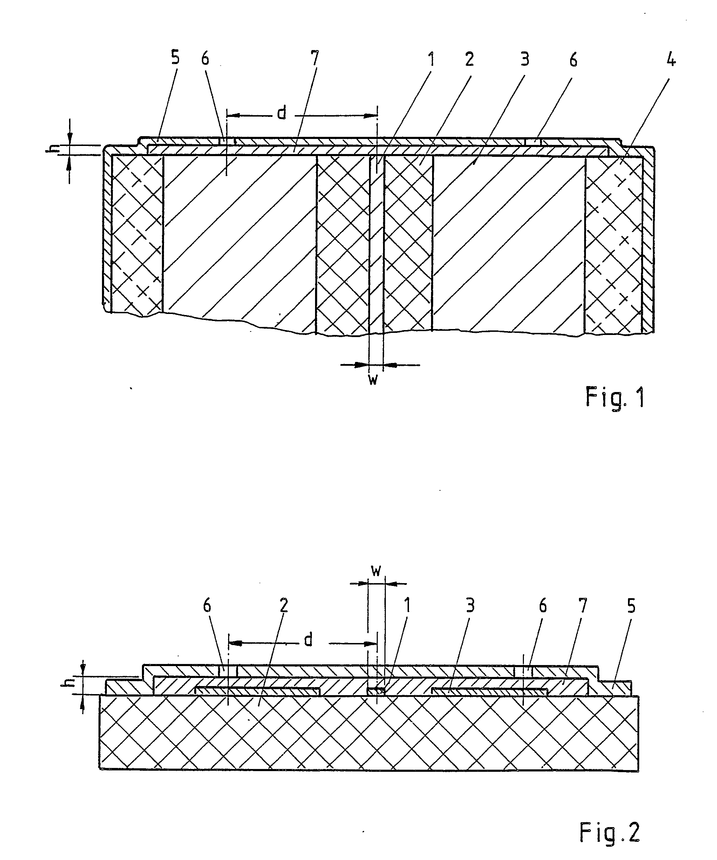

Figure 1 shows only those portions of a Clark Cell which are of interest in connection with the present invention. Further details can be taken from US—A—29 13 386. A cathode wire 1 is surrounded by an insulating rod 2, which in turn is surrounded by a hollow cylinder 3 forming the anode. A further hollow cylinder 4 of insulating material protects the anode. The entire system is covered by a non-porous hydrophobic membrane 5 which is provided with several through-holes 6. Located between the front surfaces of the cathode 1 and the anode 3 on the one side and the membrane 5 on the other side is a layer 7 of hydrophylic polymer which in the shown embodiment covers the front surfaces of both electrodes 1 and 3. For activating the cell, electrolyte has to be brought into contact with cathode 1 and the anode 3. Instead of a wire a cylinder or other cathode structure of small diameter and large length may be used.

When using the cell, the front surface of membrane 5 is exposed to the biological medium so that the electrolyte component of the medium can pass through the holes 6 and through the hydrophylic polymer layer 7 into contact with the electrodes. In this way the cell is activated. It must be prevented, however, that the direct access of oxygen through the holes 6 results in a change or increase of the oxygen diffusion through the selectively permeable membrane 5 to the electrodes. For this reason the distance (d) of hole 6 from the cathode 1 is by far larger, for instance five times larger than the width (w) of the active front surface area of cathode 1. The thickness (h) of the hydrophylic polymer layer 7 is very small, preferably less than 4 μ. Most or essentially all of the oxygen reaching cathode 1, therefore, stems from diffusion through membrane 5 and the oxygen portion entering through hole 6 and traveling along hydrophylic polymer layer 7 can be neglected. This layer may be made of a hydrogel such as polyvinyl alcohol, polyvinyl pyrrolidon, polyacrylamiden, hydroxyethylmetacrylate or derivates of these compounds. The diameter of the hole 6 is small compared with distance d. The cathode preferably is made of noble metal such as gold and the anode may consist of silver. The hydrophylic polymer acts as a sieve which is permeable for the electrolyte, oxygen, carbon dioxide and relatively small biological organic molecules such as glucose, but it is impermeable for large biological molecules such as proteins.

In the second embodiment shown in Figure 2 a silicon substrate supports cathode 1 and anode 3 which are deposited on the substrate in the known manner of forming printed or integrated circuits. Both electrodes again are covered by a layer 7 of hydrophylic polymer which is protected on the outside by means of a membrane 5. Hole 6 within the said membrane permits access of the electrolyte to the hydrophylic polymer layer so that the electrolyte can pass along this layer to cathode 1 for activating the cell. Oxygen or any other constituent of the fluid under examination diffuses through membrane 5 and layer 7 to cathode 1, therewith influencing the electro-chemical process between electrodes 1 and 3. These electrodes are connected to a source of DC and the amount of current induced by the reduction and oxidation process at the cathode and the anode, respectively, is a measure for determining the content of oxygen within the biological medium into which the sensor is inserted. Instead of silicon another insulating substrate such as glass or ceramics may be used. Silicon is preferred if the cell is produced by the processes of manufacturing integrated circuits. If thin film or thick film technology is used for making the cell, the substrate may consist of glass or ceramics.

## Claims

1. Electro-chemical cell for determining a particular property or component of a fluid comprising a cathode (1) having a width dimension (w) and spaced therefrom an anode (3), whereat a space engaging said electrodes is adapted for being filled with an electrolyte, a selectively permeable non-porous membrane (5) separating said electrodes and said space from the fluid and a thin layer (7) of hydrophilic polymer positioned between said membrane (5) and said anode (3) and cathode (1) which covers at least the cathode (1) characterized in that said membrane (5) has at least one hole (6) in it for allowing an electrolytic component of the fluid to enter said hydrophilic polymer layer (7) and in that the distance (d) between the at least one hole (6) and the cathode (1) is at least five times larger than the width (w) of the cathode (1).

2. Cell according to claim 1, characterized in that both the cathode (1) and the anode (3) are covered with the layer (7) of hydrophylic polymer.

3. Cell according to claim 1, characterized in that the hydrophylic polymer layer (7) comprises a hydrogel such as polyvinyl alcohol, polyvinyl pyrrolidon, polyacrylamiden, hydroxyethylmetacrylate and derivates of these compounds.

4. Cell according to one of claims 1 to 3, characterized in that the membrane (5) is made of a hydrophobic polymer.

5. Cell according to one of claims 1 to 4, characterized in that it is an oxygen sensor of the Clark type.

6. Cell according to one of the claims 1 to 5, characterized in that it is an ion-selective sensor.

7. Cell according to one of Claims 1 to 6, characterized in that the hydrophylic polymer layer (7) is less than 4 µ thick.

8. Cell according to one of claims 1 to 7, characterized in that an insulating substrate (2) supports the electrodes (1, 3), said insulating substrate being made of silicon, glass or ceramics.

9. Method for making a cell according to one of the claims 1 to 7, characterized in that the hydrophylic polymer layer (7) and/or the hydrophobic polymer membrane (5) are deposited on an insulating substrate (2) supporting the electrodes (1, 3) by thick film technology.

## Patentansprüche

1. Elektrochemische Zelle zum Bestimmen einer bestimmten Eigenschaft oder eines Bestandteils eines Fluids mit einer Kathode (1) von einer Breitenausdehnung (w) und im Abstand hiervon einer Anode (3), wobei ein diese Elektroden berührender Raum mit einem Elektrolyten gefüllt werden kann, ferner mit einer selektiv durchlässigen, nicht porösen Membran (5), welche die beiden Elektroden und den genannten Raum, vom Fluid trennt, und weiterhin mit einer dünnen Schicht (7) eines hydrophilen Polymers zwischen der Membran (5) und der Anode (3) und der Kathode (1), welche zumindest die Kathode (1) bedeckt, dadurch gekennzeichnet, daß die Membran (5) wenigstens ein Loch (6) aufweist für den Zutritt eines elektrolytischen Bestandteils des Fluids zu der hydrophilen Polymerschicht (7) und daß der Abstand (d) zwischen dem wenigstens einen Loch (6) und der Kathode (1) mindestens fünfmal größer ist als die Breite (w) der Kathode (1).

2. Zelle nach Anspruch 1, dadurch gekennzeichnet, daß sowohl die Kathode (1) als auch die Anode (3) mit der hydrophilen Polymerschicht (7) bedeckt sind.

3. Zelle nach Anspruch 1, dadurch gekennzeichnet, daß die hydrophile Polymerschicht (7) ein Hydrogel enthält, wie Polyvinyl-Alkohol, Polyvinyl-Pyrrolidon, Polyacrylamiden, Hydroxyäthylmetacrylat und Derivate dieser Verbindungen.

4. Zelle nach einem der Ansprüche 1 bis 3, dadurch gekennzeichnet, daß die Membran (5) aus einem wasserabweisenden Polymer besteht.

5. Zelle nach einem der Ansprüche 1 bis 4, dadurch gekennzeichnet, daß sie ein Sauerstoffühler von Clark-Typ ist.

6. Zelle nach einem der Ansprüche 1 bis 5, dadurch gekennzeichnet, daß sie ein ionenselektiven Fühler ist.

7. Zelle nach einem der Ansprüche 1 bis 6, dadurch gekennzeichnet, daß die hydrophile Polymerschicht (7) weniger als 4 µm dick ist.

8. Zelle nach einem der Ansprüche 1 bis 7, dadurch gekennzeichnet, daß ein isolierendes Substrat (2) die Elektroden (1, 3) trägt und aus Silizium, Glas oder Keramik besteht.

9. Verfahren zur Herstellung einer Zelle nach einem der Ansprüche 1 bis 7, dadurch gekennzeichnet, daß die hydrophile Polymerschicht (7) und/oder die wasserabstoßende Polymermembran (5) auf einem isolierenden, die Elektroden (1, 3) tragenden Substrat (2) durch Dickfilmtechnik aufgebracht wird.

## Revendications

1. Cellule électrochimique servant à déterminer une propriété pariculière ou un constituant particulier d'un fluide, comprenant une cathode (1) possédant une dimension en largeur (w) et une anode (3) distante de cette cathode, où un espace en contact avec ces électrodes est aménagé pour être rempli d'un électrolyte, une membrane non poreuse (5), perméable de façon sélective, séparant lesdites électrodes et ledit espace vis-à-vis du fluide, une couche mince (7) constituée en un polymère hydrophile disposée entre ladite membrane (5), ladite anode (3) et ladite cathode (1), qui recouvre au moins la cathode (1), caractérisée en ce que ladite membrane (5) comporte au moins un trou (6) permettant à un constituant électrolytique du fluide de pénétrer dans ladite couche polymère hydrophile (7), et que la distance (d) entre au moins un trou (6) et la cathode (1) est égale au moins au quintuple de la largeur (w) de la cathode (1).

2. Cellule selon la revendication 1, caractérisée en ce que la cathode (1) et l'anode (3) sont toutes deux recouvertes par la couche (7) du polymère hydrophile.

3. Cellule selon la revendication 1, caractérisée en ce que la couche de polymère hydrophile (7) comporte un hydrogel comme par exemple de l'alcool polyvinylique, de la polyvinylpyrrolidone, du polyacrylamide, de l'hydroxyéthylméthacrylate et des dérivés de ces composés.

4. Cellule selon l'une des revendications 1 à 3, caractérisée en ce que la membrane (5) est constituée par un polymère hydrophobe.

5. Cellule selon l'une quelconque des revendications 1 à 4, caractérisée en ce qu'il s'agit d'un capteur d'oxygène du type capteur de Clark.

6. Cellule selon l'une quelconque des revendications 1 à 5, caractérisée en ce qu'il s'agit d'un capteur sélectif d'ions.

7. Cellule selon l'une quelconque des revendications 1 à 6, caractérisée en ce que la couche de polymère hydrophile (7) possède une épaisseur inférieure à 4 μm.

8. Cellule selon l'une quelconque des revendications 1 à 7, caractérisée en ce qu'un substrat isolant (2) supporte les électrodes (1, 3), ledit substrat isolant étant constitué par du silicium, du verre ou une céramique.

9. Procédé pour fabriquer une cellule selon l'une quelconque des revendications 1 à 7, caractérisée en ce que la couche de polymère hydrophile (7) et/ou la membrane en polymère hydrophobe (5) sont déposées sur un substrat isolant (2) supportant les électrodes (1, 3), au moyen d'une technologie par couches épaisses.

Fig. 1

Fig.2